# EUROPEAN PATENT SPECIFICATION

(11) **EP 4 258 989 B1**
(45) Date of publication and mention of the grant of the patent: **15.07.2026**
(21) Application number: 21904271.0
(22) Date of filing: 07.12.2021
(51) Int. Cl.: A61B 5/11, A61C 5/77, A61C 7/00, A61C 9/00, A61C 11/00, A61C 13/00

(54) **SYSTEM AND METHOD FOR GENERATING A DENTAL TREATMENT PLAN**
SYSTEM UND VERFAHREN ZUR ERZEUGUNG EINES ZAHNBEHANDLUNGSPLANS
SYSTÈME ET PROCÉDÉ DE GÉNÉRATION D'UN PLAN DE TRAITEMENT DENTAIRE

(30) Priority: 10.12.2020 US 202063199158 P
(43) Date of publication of application: 18.10.2023
(73) Proprietor: Hultgren Dental Technologies, LLC, Victoria, MN 55386 (US)
(72) Inventor: HULTGREN, Bruce, Willard, Victoria, MN 55386 (US)
(74) Representative: Ström & Gulliksson AB
(86) International application number: PCT/US2021/062250
(87) International publication number: WO 2022/125577

(56) References cited:
- US-A1- 2013 066 598
- US-A1- 2019 255 778
- US-A1- 2019 290 408
- US-A1- 2020 237 486
- US-A1- 2020 297 245

## Description

### BACKGROUND

Dental procedures are often performed to adjust a patient's dentition. Examples of common adjustments that are made include installing dental restorations (including dental implants, crowns, and dentures) and orthodontic alignment of the teeth (such as using braces).

Whenever an adjustment is made to the teeth, it is important for the dentist to avoid making changes that will cause interference with teeth of the opposing dentition. Human beings have independent control of jaw motion in six degrees of freedom (horizontal position, vertical position, lateral position, sagittal plane orientation (pitch), orientation in the frontal plane (roll), and coronal plane orientation (yaw)). In both speech and mastication, jaw motion involves a combination of rotation and translation, making it difficult to accurately predict and avoid interference when creating a dental restoration or adjustment treatment plan. For example, if the adjustment results in a tooth or restoration that extends an excess vertical distance or is contoured incorrectly, the opposing teeth will bump into it when the patient bites down. As a result, efforts are made to avoid introducing such interference. Due to the complexity of jaw movement, and the fact that each person has a unique jaw structure and dentition, current techniques often rely on trial and error to eliminate interference after an adjustment is made.

Furthermore, even with efforts to avoid introducing interference, unexpected changes can occur in the patient's jaw motion as a result of adjustments made in the patient's dentition. Such changes can also result in undesirable interference.

Relevant prior art is exemplified by US 2013/066598 A1, US 2020/237486 A1, US 2019/290408 A1, US 2020/297245 A1, and US 2019/255778 A1.

### SUMMARY

In general terms, this disclosure is directed to a data processing system for developing a dental treatment plan. In particular, this disclosure relates to a data processing system and computer-implemented method of obtaining jaw motion data and analyzing the motion data against a database of existing jaw motion data to generate a dentition treatment plan.

In one possible configuration and by non-limiting example, the system is used to approximate motion of a patient's jaw by comparing basic jaw measurements to a parametric model of known human jaw measurements. In such a configuration, a dentist or technician would first obtain basic measurements of a patient's jaw structure and dentition. The system further includes a parametric model of known human jaw measurements divided into representative groups. The system compares the measurements obtained from the patient against the parametric model to determine which of the representative groups is most similar to the patient's jaw structure. Based on the identification of the most similar representative group, the system can accurately predict the motion of the patient's jaw and dentition without having to take detailed scans or measurements of the patient. The predictive motion is used to create a treatment plan. In some examples, this process could be used to efficiently and inexpensively design crowns or similar dental restorations with greater precision compared to existing techniques, but without the expense of investing in a precision scanner and modeling software.

One aspect is a method of analyzing a dental treatment plan that includes the steps of: performing a measurement of a patient's jaw structure and dentition; providing a parametric model of known human jaw measurements, wherein the parametric model is divided into representative groups; comparing the measurement of the jaw structure and dentition to the parametric model; identifying which of the representative groups is most similar to the patient's jaw structure and dentition; and determining a treatment plan with dentition adjustment based on the identified representative group. In certain embodiments, the method may include predicting a motion adjustment based on the treatment plan.

Another aspect is a system of creating a dental treatment plan that includes performing a measurement of a patient's jaw structure and a dental treatment plan generator that generates a treatment plan based on the measurements of the patient's jaw structure by identifying a representative group from a parametric model of known human jaw measurements that is most similar to the patient's jaw structure. The representative group may also include interference boundary information that is used by the system to generate a treatment plan that is unlikely to result in interference in the patient's dentition.

These and other aspects and embodiments are described in greater detail below, in reference to the attached drawing figures.

### BRIEF DESCRIPTION OF THE DRAWINGS

FIG. 1 is a flow chart illustrating an example method of analyzing a dental treatment plan.
FIG. 2 is a schematic block diagram illustrating an example patient evaluation system for analyzing a dental treatment plan.
FIG. 3 is a block diagram illustrating an example treatment plan generator of a treatment plan generation system.
FIG. 4 illustrates an example architecture of a computing device, which can be used to implement aspects according to the present disclosure.
FIG. 5 is a flow chart illustrating an example method of predicting a motion adjustment responsive to a restoration preparation.
FIG. 6 is a flow chart illustrating an example method of predicting a motion adjustment responsive to a dental alignment plan.
FIG. 7 is a flow chart illustrating an example method of generating a dental treatment plan.
FIG. 8 is a schematic diagram illustrating representative segments of a dataset of jaw data.
FIG. 8A is another schematic diagram illustrating representative segments of a dataset of jaw data.
FIG. 9 is a flow chart illustrating an example method of generating a dental treatment plan.
FIG. 10 is a block diagram illustrating an example treatment plan generator of a treatment plan generation system.
FIG. 11 is a block diagram illustrating an example treatment plan generator of a treatment plan generation system.
FIG. 12 is a front schematic view of a human skull, showing the jaw in a closed position.
FIG. 13 is a side schematic view of the human skull of FIG. 12, showing the jaw in the closed position.
FIG. 14 is another side schematic view of the human skull of FIG. 12, showing the motion of the jaw.
FIG. 15 is a schematic view showing measurements that could be used to develop a patient jaw profile.

### DETAILED DESCRIPTION

The present disclosure relates to a system and method for generating a dental treatment plan that minimizes potential interference between teeth without the need to take detailed scans of a patient's dentition and jaw structure.

A patient's dentition consists of teeth of both upper and lower dental arches on the upper and lower jaw, respectively. Certain dental procedures performed by a dentist on the patient involve adjusting a tooth or teeth of one or both dental arches. In some examples, the dental procedure can involve occlusal equilibration. In other examples, the dental procedure can involve dental restorations, including dental implants, bridges, dentures (full and partial), and/or crowns. In further examples, the dental procedure can involve orthodontic alignment of the teeth, including use of braces. In yet further examples, the dental procedure can involve surgical adjustments, including implant-supported denture surgeries and orthognathic surgeries. Moreover, the dental procedure can involve a combination of occlusal equilibration, dental restorations, orthodontic alignment of teeth, and surgeries.

Whenever an adjustment is made to a tooth, there is a potential that the adjustment will cause interference with teeth of the opposing dental arch. For example, if the adjustment results in the tooth being extruded (e.g., extending and excess vertical distance and not matching the opposing teeth vertically), an interference will occur between the tooth and the opposing teeth when the patient bites down. Additionally, changes can occur in the patient's jaw motion as a result of the adjustment made to the tooth. For example, prior to the dental procedure, the patient's dentition may already have some interference that has caused the patient's jaw motion to be restricted, such as a shape of the tooth or the way in which the tooth fits together with an opposing tooth that restricts the jaw to only move so far vertically, anteroposteriorly, and transversely (e.g., up and down, front and back, and side to side). In some situations, the interference is removed as a result of the adjustment to the tooth, causing the jaw motion to adjust and have a less restricted, more extensible movement in one or more directions, which in turn causes the teeth to move relative to one another in different ways. For example, a particular tooth can now move further vertically, anteroposteriorly, or transversely (e.g., further up or down, front or back, or side to side), which changes the way the tooth fits or interacts with teeth of the opposing dental arch, and can cause an interference that affects the patient's bite or chewing.

Often times, when generating and performing a dental treatment plan that involves a dentition adjustment, a dentist only takes into account and proactively avoids interferences with opposing teeth directly created by the dentition adjustment, and does not consider subsequent jaw motion changes and corresponding effects. As a result, when the dentition is adjusted, a corresponding jaw motion adjustment can occur that was not accounted for in the dental treatment plan. Even if the dentist performs a scan post-procedure, this scan is static (e.g., comprised of multiple static bites) and may not capture interferences created from the jaw motion adjustment. Accordingly, the patient may leave the dentist office unaware, but as the patient more dynamically moves their jaw (e.g., when chewing food), the patient may then notice and experience discomfort or pain due to the interference requiring the patient to return to the dentist for a correction. Once the interference is identified, the patient will need to spend additional time at the dentist office for the dentist to modify the preparation and/or materials used for the dental procedure, and may still need to come back for a later visit if the dentist needs to order new materials (e.g., a new crown) based on the changes caused by the interference. In other scenarios, the motion of the jaw may change slowly over time as a result of the dentition adjustment causing a new interference. Similarly, additional visits to the dentist office will be required once effects of the new interference are noticed by the patient in order to identify and correct the new interference.

To proactively avoid interferences caused by both dentition adjustment and jaw motion adjustment and prevent a patient from enduring multiple unnecessary office visits, embodiments described herein can be implemented to predict a motion adjustment responsive to a dental treatment plan including a dentition adjustment. The dental treatment plan can then be modified to accommodate the predicted motion adjustment prior to performance of the dentition adjustment. Additionally, data collected to predict the motion adjustment, such as scans of the patient's dentition, motion data from dentition motion assessments, and various digital models generated therefrom, and data associated with the predicted motion adjustment, such as interference boundaries, can be stored for use and/or reference in future dental procedures.

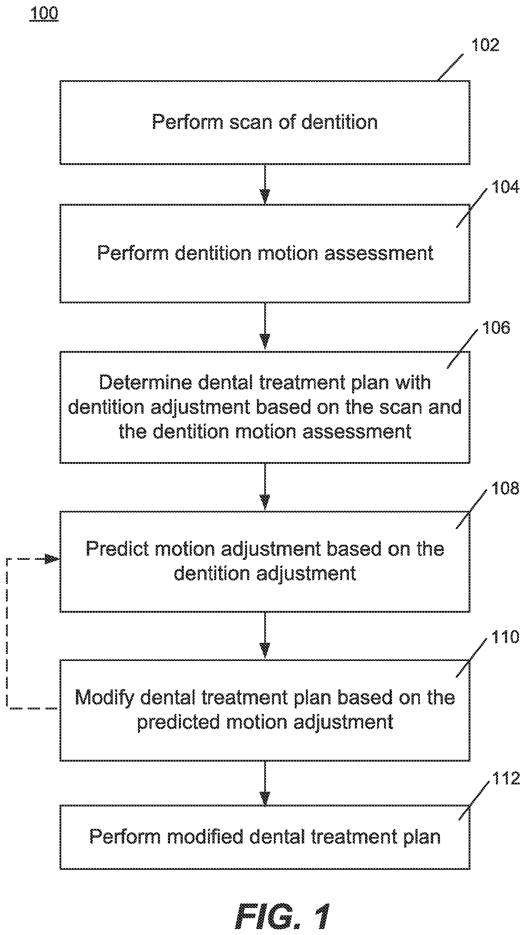

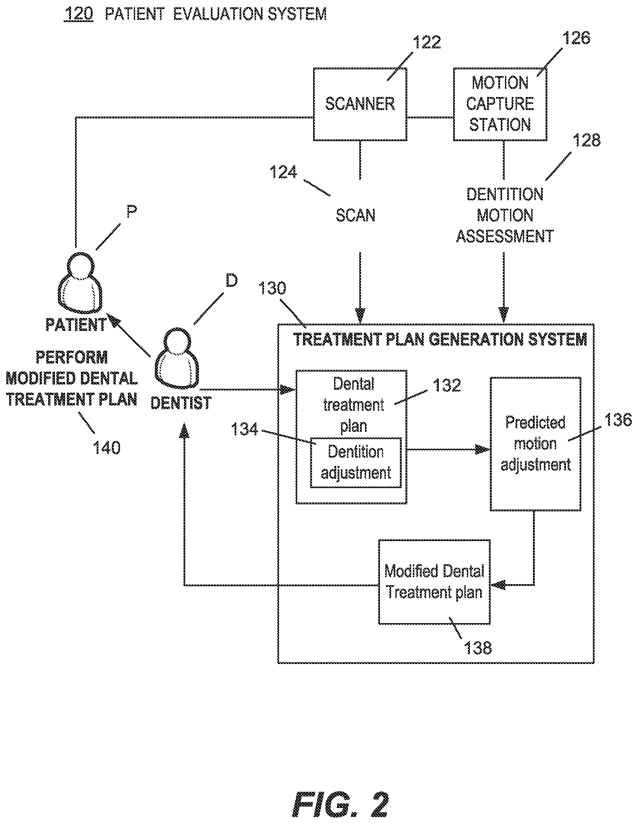

FIG. 1 is a flow chart illustrating a prior art method 100 of analyzing a dental treatment plan. The method 100 can be implemented, for example, by the example patient evaluation system 120 described herein with reference to FIGS. 2-3 below. In this example, the method 10 includes operations 102, 104, 106, 108, 110, and 112.

Prior to performing a dental procedure, a scan of a patient's dentition is performed at operation 102. The scan identifies a position and an orientation of each tooth in the upper and lower dental arches of the patient's dentition with respect to each other tooth.

At operation 104, a dentition motion assessment is performed. The dentition motion assessment captures motion data representing the movement of the patient's dental arches relative to each other. For example, the dentition motion assessment captures how each tooth in the upper and lower dental arches move with respect to one another.

In some embodiments, a pre-treatment interference boundary can be determined utilizing the position and orientation of each tooth in the dental arches provided by the scan and the movement of each tooth in the dental arches provided by the motion data. The pre-treatment interference boundary is a boundary of function between teeth of opposing dental arches before a treatment plan has been implemented. The pre-treatment interference boundary is defined by the interface or interaction between the teeth on the opposing dental arches. For example, the pre-treatment interference boundary indicates how far any of the teeth can move vertically, anteroposteriorly, and transversely. If a tooth extends or crosses over the pre-treatment interference boundary in one or more planes, an interference with teeth of the opposing dental arch of the dentition is created.

To provide additional context, the pre-treatment interference boundary may be analogous to a functionally generated pathway (FGP) of occlusion. An FGP is generated based on registered paths of movement of occlusal surfaces of the teeth of one dental arch, to the teeth or occlusion rims of the opposing dental arch that are recorded using a medium. An example technique for generating an FGP includes adapting a material over the occlusal surface of a tooth or teeth and having the patient occlude the teeth in the intercuspal position and move the lower jaw throughout all excursions. This allows the opposing teeth to three-dimensionally record border movements in each jaw position in the material to define a boundary of function between teeth of opposing dental arches. However, the pre-treatment interference boundary may be determined more quickly and accurately (and with less inconvenience to the patient) based on the position and orientation of each tooth in the dental arches provided by the scan and the movement of each tooth in the dental arches provided by the motion data.

An interference boundary can be thought of as a surface that would be generated if a tray, containing a pliable material such as paste or gel, was mounted to one of the dental arches, and then the patient were to bite down into the material and move the opposing dentition around in all directions. The result would be a surface that records the furthest extent that the teeth can reach. For example, a surface that records perimeters of a boundary defining a vertical, anteroposterior, and traversal reach of the teeth. This same or similar type of an interference boundary can be generated and stored digitally.

At operation 106, a dental treatment plan with a dentition adjustment is determined based on the scan and the dentition motion assessment. The dentition adjustment can involve any kind of change in the structure or positions of the teeth. To provide some examples, the dentition adjustment can include a removal, re-shaping, and/or re-alignment of a portion or entirety of a tooth or teeth on one or both dental arches of the patient's dentition. When determining the dental treatment plan, the adjustment to a tooth or teeth of one dental arch of the dentition is defined to avoid an interference with teeth of the opposing dental arch of the dentition. For example, the scan performed at operation 102 and the motion data obtained from the dentition motion assessment performed at operation 104 are utilized to visualize a position and orientation of a tooth associated with the dentition adjustment and how the tooth can move vertically, anteroposteriorly, and transversely to ensure that the dentition adjustment does not move or adjust the tooth beyond the confines of the pre-treatment interference boundary (e.g., does not violate the pre-treatment interference boundary to avoid creating an interference).

However, even with efforts to avoid introducing interference by accounting for the dentition adjustment, changes can occur in the patient's jaw motion as a result of adjustments made to the patient's dentition. Additionally or alternatively, changes in the patient's jaw motion can be caused by changes to joints of the jaw (e.g., as a result of surgery). Such changes in jaw motion can also result in undesirable interference. Therefore, to further bolster efforts to avoid introducing interference, the changes occurring to the patient's jaw motion as a result of the dentition adjustment can also be accounted for by predicting a motion adjustment based on the dentition adjustment at operation 108. For example, a determination is made as to whether an interference will be removed after the dentition adjustment (e.g., whether an already existing interference within patient's dentition due to a shape of a tooth or current interactions between teeth will be removed) and, if so, a determination of associated effects of the interference removal (e.g., a determination of changes to the jaw motion) is made to predict the motion adjustment. In some examples, because the jaw's range of motion influences how far a tooth or teeth may be able to move in direction and magnitude, the associated effects of the removal can cause a change to the pre-treatment interference boundary. Thus, a post-dentition adjustment interference boundary is predicted to represent the interference boundary following the dentition adjustment and removal of the interference.

At operation 110, the dental treatment plan is modified based on the predicted motion adjustment. For example, if it is determined that the dentition adjustment proposed by the dental treatment plan will cause changes to the jaw motion, the dental treatment plan is modified to accommodate the predicted motion adjustment so that the dental treatment plan continues to avoid introducing any interference. As one example, a jaw motion change may result in a change to the pre-treatment interference boundary. For example, the jaw may now be more or less extensible, and the boundary may change accordingly. Therefore, the dental treatment plan is modified to ensure that the dentition adjustment made does not go beyond the confines of or violate the predicted post-dentition adjustment interference boundary. Accordingly, the modified dental treatment plan proactively accounts for (e.g., in order to avoid) interferences caused by both the dentition adjustment and subsequent motion adjustment of the jaw.

In some embodiments the operations 108 and 110 can be repeated one or more times until interference is eliminated, or until the interference is less than a predetermined amount. For example, the predetermined amount is less than a predetermined overlap distance. Once the interference is eliminated, or the interference is less than the predetermined amount, the modified dental treatment plan can be performed at operation 112.

FIG. 2 is a schematic block diagram illustrating an example patient evaluation system 120 for implementing the method 100 of analyzing a dental treatment plan described in FIG. 1. The patient evaluation system 120 includes a scanner 122, a motion capture station 126, and a treatment plan generation system 130.

Prior to a dentist D performing a dental procedure on a patient P, a scan 124 of dentition of the patient P is captured by the scanner 122 to perform operation 102 and a dentition motion assessment 128 is performed on patient P by the motion capture station 126 to perform operation 104. The scan 124 and dentition motion assessment 128 are provided as input to the treatment plan generation system 130. Examples of the motion capture station 126 are described in U.S. Publication No. 2017/0312065, filed on April 28, 2017, and titled DETERMINING JAW MOVEMENT, which is referred to herein, and specifically the description of the motion capture station 126. For example, the motion capture station 126 may comprise a patient assembly that includes a clutch to be worn by the patient P on a dentition of the patient P. The clutch includes a dentition coupling device to couple to the dentition of the patient P and a position indicating system rigidly connected to the dentition coupling device, where the position indicating system emits a plurality of light beams. The motion capture station 126 can also include an imaging system and a motion determining device, where the imaging system captures a plurality of image sets that each include at least one of a plurality of screens upon which the light beams project, and the motion determining device processes the captured image sets to determine the motion of the patient's dentition.

The scan 124 provides a position and an orientation of each tooth in the upper and lower dental arches of the dentition with respect to each other tooth. In certain embodiments, scan 124 may also include soft tissue data such as the patient's lip profile. If soft tissue data is being scanned, it may also be desirable to obtain scans of the patient at rest and smiling, which would allow the treatment plan generation system 130 to identify the relationship between the patient's teeth and soft tissue (lips) in three dimensions. That relationship, particularly between the patient's upper front teeth and the lips would enhance the system's 130 ability to provide design flexibility when generating a dental treatment plan.

The dentition motion assessment 128 comprises motion data providing the movement of the dental arches relative to each other, including how each tooth in the upper and lower dental arches move with respect to one another. In some examples, the dentition motion assessment 128 also provides a pre-treatment interference boundary. In other examples, the treatment plan generation system 130 determines the pre-treatment interference boundary from data associated with the scan 124 and the dentition motion assessment 128. The pre-treatment interference boundary is a boundary of function between teeth of opposing dental arches before a treatment plan has been implemented, and is defined by the interface or interaction between the teeth on the opposing dental arches. For example, the pre-treatment interference boundary indicates how far each particular tooth can move vertically, anteroposteriorly, and transversely. If a tooth extends or crosses over the pre-treatment interference boundary in one or more planes, an interference with teeth of the opposing dental arch of the dentition is created.

Based on the data associated with the scan 124 and dentition motion assessment 128, the treatment plan generation system 130 determines a dental treatment plan 132 that includes a dentition adjustment 134 to perform operation 106. The dentition adjustment 134 can involve any kind of change in the structure or positions of the teeth, such as a removal, re-shaping, and/or re-alignment of a portion or entirety of a tooth or teeth on one or both dental arches of the patient P's dentition, among other examples. When determining the dental treatment plan 132, the dentition adjustment 134 is defined to avoid an interference between a tooth or teeth associated with the dentition adjustment 134 and teeth of the opposing dental arch of the dentition. For example, data from the scan 124 and the dentition motion assessment 128 are utilized to visualize a position and orientation of a tooth or teeth associated with the dentition adjustment 134 and how the tooth or teeth can move vertically, anteroposteriorly, and transversely to ensure that the dentition adjustment does not move or adjust the tooth or teeth beyond the confines of the pre-treatment interference boundary (e.g., does not violate the pre-treatment interference boundary to avoid introducing an interference).

However, because the dental treatment plan 132 includes the dentition adjustment 134 and changes can occur in the patient's jaw motion as a result of the dentition adjustment 134, the treatment plan generation system 130 further determines a predicted motion adjustment 136 based the dentition adjustment 134 to perform operation 108. For example, a determination is made as to whether an interference will be removed after the dentition adjustment 134 and if so, a determination of associated effects of the removal of the interference (e.g., a determination of changes to the jaw motion) is made to determine the predicted motion adjustment 136. In some examples, because the jaw's range of motion influences how far a tooth or teeth may be able to move in direction and magnitude, the associated effects of the removal of the interference can cause a change to the pre-treatment interference boundary. Thus, a post-dentition adjustment interference boundary is predicted to represent the interference boundary following the changes resulting from the removal of the interference.

The treatment plan generation system 130 then uses the predicted motion adjustment 136 to modify the dental treatment plan 132 to create a modified dental treatment plan 138 to perform operation 110. For example, if it is determined that the dentition adjustment 134 of the dental treatment plan 132 will cause changes to the jaw motion, the dental treatment plan 132 is modified to accommodate the predicted motion adjustment so that the dental treatment plan 132 continues to avoid introducing any interference. As one example, a jaw motion change may result in a change to the pre-treatment interference boundary. For example, the jaw may now be more or less extensible, and the boundary may change accordingly. Therefore, the dental treatment plan 132 is modified to the modified dental treatment plan 138 to accommodate the predicted motion adjustment 136. For example, the modified dental treatment plan 138 ensures that the dentition adjustment 134 made does not go beyond the confines of or violate the predicted post-dentition adjustment interference boundary.

To perform operation 112, the treatment plan generation system 130 provides the modified dental treatment plan 138 as output to the dentist D such that the dentist D performs 140 the modified dental treatment plan 138 that proactively avoids introducing interferences potentially caused by both the dentition adjustment 134 and the predicted motion adjustment 136.

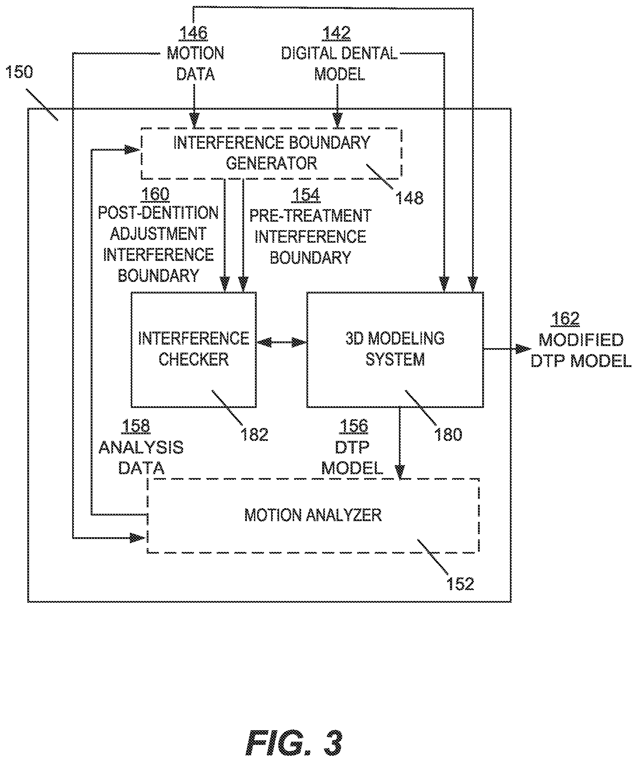

FIG. 3 is a block diagram illustrating an example of the treatment plan generator 150 of the treatment plan generation system 130 described in FIG. 3. The treatment plan generator 150 includes one or more components, including at least a three-dimensional (3D) modeling system 180 and an interference checker 182. In some embodiments, treatment plan generator 150 may also include the interference boundary generator 148 and the motion analyzer 152. In other embodiments, the interference boundary generator 148 and the motion analyzer 152 are separate components or subsystems of the treatment plan generation system 130.

The treatment plan generator 150 determines the dental treatment plan 132. The dental treatment plan 132 includes at least one dentition adjustment 134 for the patient P. Typically, the treatment plan generator 150 operates in cooperation with an operator user, such as the dentist D, to generate the dental treatment plan 132. In some examples, the dental treatment plan 132 is three-dimensional digital data that represents a dental treatment plan (DTP) model 156.

In some embodiments, the 3D modeling system 180 of the treatment plan generator 150 includes computer-aided-design (CAD) software that generates a graphical display of a digital dental model 142 representing dentition of the patient P before the treatment and, either automatically or in cooperation with an operator user, such as the dentist D, generates the DTP model 156 that identifies at least one dentition adjustment 134 to be made from the original digital dental model 142. Example software that can be implemented by the 3D modeling system 180 include 3Shape CAD/CAM software, Align technology iTero software, Orchestrate Orthodontic Technologies Orchestrate 3D Treatment Planning software, Forestadent Onyx software, and D4D Technologies E4D Dental System, uLab Systems dental aligner planning software, among other similar software. In some embodiments the operator interacts with and manipulates the DTP model 156 to define the dentition adjustment 134 to be made. Additionally, in other embodiments, the 3D modeling system 180 partially or fully automates the generation of the DTP model 156.

In further embodiments, the 3D modeling system 180 can include surgical software that is able to determine a new interference boundary (e.g., the post dentition adjustment interference boundary 160) based on changes to a horizontal and/or vertical position of one or both jaws (e.g., a realignment of the jaws) as a result of various types of surgeries. As one example, when performing a surgery for implant-supported dentures, a portion of the bone is removed from each jaw (e.g., about 0.5 cm from each jaw), which could change the vertical dimension of the jaw. Therefore, a new interference boundary based on the change in vertical dimension can be determined by the software and used to create provisional and/or permanent restorations to be attached to the surgical implant. As another example, when an orthognathic surgery (e.g., corrective jaw surgery) is performed in conjunction with preceding and subsequent dental alignments (e.g., braces), the interference boundary can change as the dimensions of the jaw change. Therefore, multiple new interferences boundaries based on the various stages of change in dimension can be determined by the software (e.g., a boundary post-dental alignment and pre-surgery, a boundary post-surgery, and a boundary post-dental alignment).

In further embodiments, the 3D modeling system 180 comprises digital tools that mimic the tools used by laboratory technicians. Example tools include definition tools, movement tools, alignment tools, segmentation tools, measurement tools, and preparation tools, among other similar tools. In some embodiments, these tools enable a user, such as the dentist D, to interact with and manipulate the digital dental model 142 in accordance with the motion data 146 to develop the dental treatment plan 132 and define the dentition adjustment 134 within the DTP model 156. In other embodiments, the tools may be automated (e.g., do not require user interaction/manipulation).

Definition tools can define the dentition adjustment 134 associated with the dental treatment plan 132. Movement tools can move the patient's dentition according to the motion data 146 (which may be similar to a physical articulator, for example). Alignment tools can be used to simulate an alignment of a tooth or teeth with adjacent and/or opposing teeth based on digital dental model 142. Segmentation tools segment the digital dental model 142 to separate each tooth from adjacent teeth, which is useful, for example, to allow individual teeth (or groups of teeth) within the digital dental model 142 to be moved relative to the other teeth. Measurement tools allow a user, such as dentist D, to perform measurements that may be needed to determine a shape, size, or type of materials that will be needed for a restoration and/or for determining the parameters for the restoration preparation (e.g., how much of and which portion of a tooth to grind, as well as how to shape). Preparation tools enable simulation of at least part of the dental procedure (e.g., a preparation for a restoration) on a tooth or teeth. For example, if the dental procedure is a crown restoration, a tooth may be cut down and shaped within the digital dental model 142 to simulate the crown preparation to be performed.

The interference checker 182 is used by the 3D modeling system 180 to aid in the definition of the dentition adjustment 134 within the DTP model 156 to avoid an adjustment that will cause interference with teeth of the opposing dentition due to a tooth that projects past a pre-treatment interference boundary 154, for example. The pre-treatment interference boundary 154 is a boundary of function between teeth of the dental arch on the upper jaw and teeth of the opposing dental arch on the lower jaw, and is defined by the interface or interaction between the teeth on the opposing dental arches before a treatment plan is implemented. In some embodiments, the interference boundary generator 148 uses the digital dental model 142 and the motion data 146 to determine and provide the pre-treatment interference boundary 154 to the interference checker 182. The interference checker 182 is then used in conjunction with the 3D modeling system 180 to ensure that the dentition adjustment 134 does not move or adjust the tooth or teeth beyond the confines of the pre-treatment interference boundary 154 (e.g., does not violate the pre-treatment interference boundary 154).

If the interference checker 182 identifies a violation of the pre-treatment interference boundary 154 as the 3D modeling system 180 and/or operator user are defining or manipulating the dentition adjustment 134 to generate the DTP model 156 from the original digital dental model 142, an alert may be provided within the graphical display of the digital dental model 142. In some embodiments, the interference checker 182 is also configured to provide color coded indications of violations directly on the graphical display of the digital dental model 142 itself to visually show the operator user where the violations are occurring to help inform the operator how to further modify or manipulate the dentition adjustment 134 to avoid the interference.

However, even with efforts to avoid introducing interference by accounting for interferences caused by the dentition adjustment 134, changes can occur in the patient's jaw motion as a result of the dentition adjustment 134. Such changes in jaw motion can also result in undesirable interference. Therefore, to further bolster efforts to avoid introducing interference, the changes occurring to the patient's jaw motion as a result of the dentition adjustment 134 can also be accounted for by predicting, by the motion analyzer 152, a motion adjustment based on the dentition adjustment 134. In some embodiments, the motion analyzer 152 analyzes the DTP model 156, along with the motion data 146, to predict what changes will occur in the patient's jaw motion as a result of the dentition adjustment 134, and then analyzes the DTP model 156 based on the predicted changes in the patient's jaw motion to generate analysis data 158.

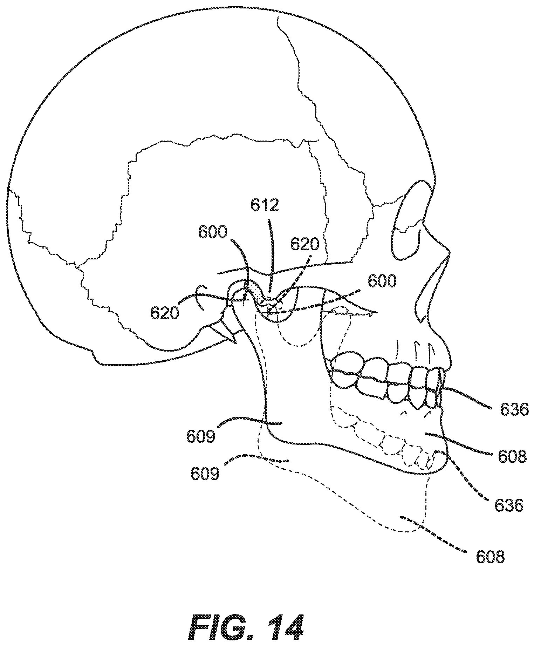

Various embodiments can be implemented by the motion analyzer 152 to predict the motion adjustment. In one or more of the embodiments, a location of a screw axis 600 of the patient's jaw 602 is determined. As shown in FIG. 14, the screw axis 600 corresponds to the condyloid process 604 of the temporomandibular joint 606 of the patient and is an axis about which the lower jaw (mandible) 608 rotates and translates along. When a patient begins opening their jaw 602, the screw axis 600 remains stationary as the jaw begins to open, resulting in pure rotational motion for the initial opening. As the patient opens their jaw 602 further, the screw axis 600 continues to rotate, but also translates along the contour of the articular fossa 612.

Accordingly, the location of the screw axis 600 imposes limitations on a range of motion of the jaw 602. Existing methods of determining the location of screw axis 600 lack precision, especially when measured from a static image, regardless of whether the image is two or three-dimensional. One aspect of the present disclosure is an improvement of the accuracy with which the location of screw axis 600 can be identified.

Mathematical coordinates of the range of motion relative to the patient's dentition can be determined and used as parameters for simulating the motion of the jaw after the dentition adjustment 134 is made. For example, the DTP model 156 that includes the dentition adjustment 134 can be used in conjunction with the screw axis location and other jaw-related parameters to simulate the motion of the jaw after the dentition adjustment 134.

Other jaw-related characteristics or parameters, such as a flexibility or "sponginess" in three different planes of space of the temporomandibular joint 606, can be determined and used as data input for simulating the motion of the jaw 602. Some non-limiting examples of j aw-related parameters include laterotrusion, laterosurtrusion, laterodetrusion, lateroretrusion, lateroprotrusion, and Bennett shift (lateral translation). Sources of data inputs for simulating the motion of the jaw 602 may include radiographic data obtained from dental cone beam CT scans. One downside to using 2-d radiographic data in the sagittal plane, is it is difficult to determine any asymmetry in the patient's jaw. Existing models of jaw motion do not account for such asymmetry. In some embodiments, a face bow or caliper may be used to measure certain aspects of the patient's jaw 602. Such measurements may include but are not limited to the distance between the patient's condyles 620, as well as identifying any rotation of the jaw 602 in the coronal plane, meaning the condyles on either side of the patient's jaw are not aligned with each other.

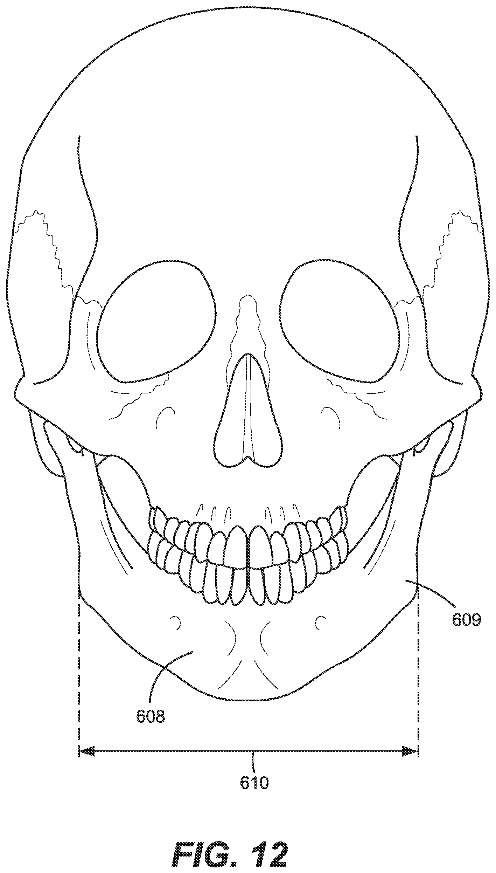

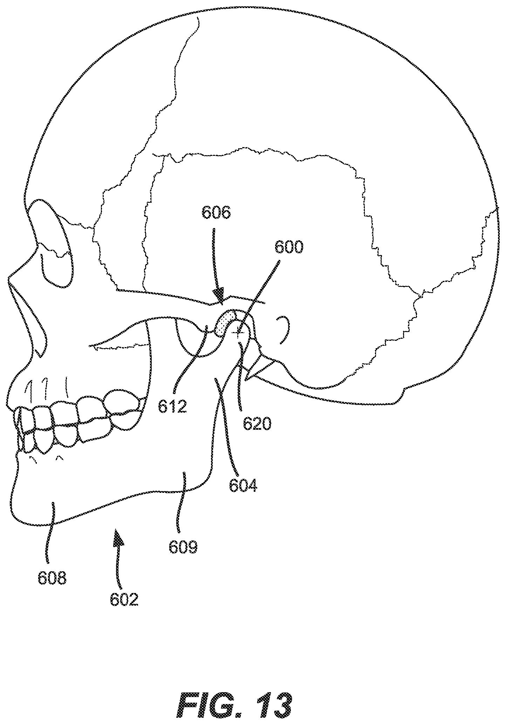

In one embodiment, the motion adjustment prediction is determined via a simulation guided by manual inputs and manipulations of a user, such as the dentist D, to a digital (virtual in-virto) articulator. The digital articulator can be displayed through a user interface of a computing device 190 and the dentist D can interact with the digital articular using input devices 216 of the computing device, such as a touch sensor 224 (such as a touchpad or touch sensitive display), a mouse 220, or a keyboard 218, among other suitable input devices. In some embodiments, the digital articulator includes the DTP model 156 defining the dentition adjustment 134, and the user can use the input devices to manually shift the lower jaw 608 (see FIGS. 12-14) of the DTP model 156 within the simulation. The lower jaw 608 is manually shifted up and down, forward and back, and side to side to collect analysis data 158 within the range of motion permitted by the screw axis 600 location and other j aw-related characteristics or parameters for determining whether an interference present in the pre-treatment dentition that restricted the motion of the jaw 602 has been removed as a result of the dentition adjustment 134 defined in the DTP model 156. For example, a post-dentition adjustment interference boundary 160 is determined by the interference boundary generator 148 based on analysis data 158 collected during the manual shifting, and can be compared to the pre-treatment interference boundary 154 to determine whether an interference has been removed.

In another embodiment, the motion adjustment prediction is determined via a simulation employing artificial intelligence to perform automated shifting of the lower jaw 608 of the DTP model 156 within the digital articulator. For example, the lower jaw 608 within the above-described simulation is automatically shifted up and down, forward and back, and side to side within the range of motion permitted by the screw axis 600 location and other jaw-related characteristics or parameters to collect analysis data 158 for determining whether an interference present in the pre-treatment dentition that restricted the motion of the jaw has been removed as a result of the dentition adjustment 134 defined in the DTP model 156. Similarly, the post-dentition adjustment interference boundary 160 is determined by the interference boundary generator 148 based on analysis data 158 collected during the automatic shifting, and can be compared to the pre-treatment interference boundary 154 to determine whether an interference has been removed.

In further embodiments, utilizing the DTP model 156 that includes the dentition adjustment 134 as well as the range of motion limitations designated by the determined screw axis location and other jaw-related characteristics or parameters, data points are recorded at a plurality of different three dimensional points within an xyz space to determine one or more directions and a magnitude thereof that each tooth of the dentition moves as the lower jaw of the DTP model 156 is moved within the xyz space to predict the post-dentition adjustment interference boundary 160. For example, a reference point may be selected within the jaw. From the reference point, a direction and magnitude in which each tooth is able to move is measured as the jaw is moved (e.g., up and down, side to side, and front to back) every n millimeters, for example. In some embodiments, the measurements are relative to a pitch, a yaw, and a roll of the jaw. The analysis data 158 comprises the recorded data points, which are utilized by the interference boundary generator 148 to determine the post-dentition adjustment interference boundary 160, which can be compared to the pre-treatment interference boundary 154 to determine whether an interference has been removed.

The 3D modeling system 180 can then modify the dental treatment plan 132 as needed to accommodate for the predicted motion adjustment (e.g., to ensure that the dentition adjustment 134 made does not go beyond the confines of or violate the predicted post-dentition adjustment interference boundary 160). The 3D modeling system 180 can correspondingly adjust the DTP model 156 to generate a modified DTP model 162. The modified DTP model 162 is then provided as output of the treatment plan generator 150.

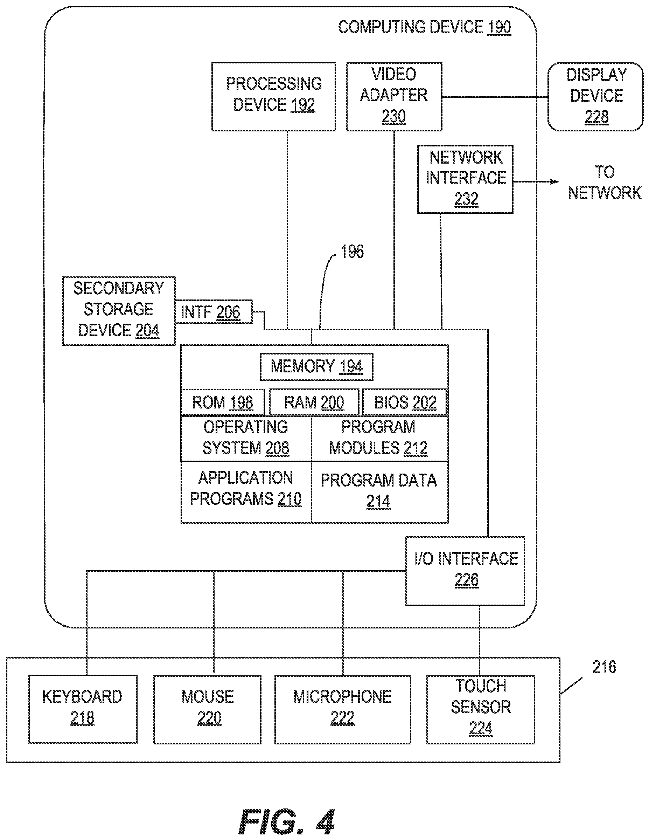

FIG. 4 illustrates an exemplary architecture of a computing device 190 that can be used to implement aspects of the present disclosure, including any of the plurality of computing devices described herein, such as a computing device of the patient evaluation system 120, the scanner 122, the motion capture station 126, the treatment plan generation system 130, the interference boundary generator 148, the treatment plan generator 150, the 3D modeling system 180, the interference checker 182, the motion analyzer 152, the rapid fabrication machine 164, the restoration fabrication station 168, or any other computing devices that may be utilized in the various possible embodiments.

The computing device illustrated in FIG. 4 can be used to execute the operating system, application programs, and software modules (including the software engines) described herein.

The computing device 190 includes, in some embodiments, at least one processing device 192, such as a central processing unit (CPU). A variety of processing devices are available from a variety of manufacturers, for example, Intel or Advanced Micro Devices. In this example, the computing device 190 also includes a system memory 194, and a system bus 196 that couples various system components including the system memory 194 to the processing device 192. The system bus 196 is one of any number of types of bus structures including a memory bus, or memory controller; a peripheral bus; and a local bus using any of a variety of bus architectures.

Examples of computing devices suitable for the computing device 190 include a desktop computer, a laptop computer, a tablet computer, a mobile computing device (such as a smart phone, an iPod^{®} or iPad^{®} mobile digital device, or other mobile devices), or other devices configured to process digital instructions.

The system memory 194 includes read only memory 198 and random access memory 200. A basic input/output system 202 containing the basic routines that act to transfer information within computing device 190, such as during start up, is typically stored in the read only memory 198.

The computing device 190 also includes a secondary storage device 204 in some embodiments, such as a hard disk drive, for storing digital data. The secondary storage device 204 is connected to the system bus 196 by a secondary storage interface 206. The secondary storage device 204 and their associated computer readable media provide nonvolatile storage of computer readable instructions (including application programs and program modules), data structures, and other data for the computing device 190.

Although the exemplary environment described herein employs a hard disk drive as a secondary storage device, other types of computer readable storage media are used in other embodiments. Examples of these other types of computer readable storage media include flash memory cards, solid state disk drives, digital video disks, compact disc read only memories, digital versatile disk read only memories, random access memories, or read only memories. Some embodiments include nontransitory media. Additionally, such computer readable storage media can include local storage or cloud-based storage.

A number of program modules can be stored in secondary storage device 204 or system memory 194, including an operating system 208, one or more application programs 210, other program modules 212 (such as the software engines described herein), and program data 214. The computing device 190 can utilize any suitable operating system, such as Microsoft Windows^{™}, Google Chrome^{™} OS, Apple OS, Unix, or Linux and variants and any other operating system suitable for a computing device. Other examples can include Microsoft, Google, or Apple operating systems, or any other suitable operating system used in tablet computing devices.

In some embodiments, a user provides inputs to the computing device 190 through one or more input devices 216. Examples of input devices 216 include a keyboard 218, mouse 220, microphone 222, and touch sensor 224 (such as a touchpad or touch sensitive display). Other embodiments include other input devices 216. The input devices are often connected to the processing device 192 through an input/output interface 226 that is coupled to the system bus 196. These input devices 216 can be connected by any number of input/output interfaces, such as a parallel port, serial port, game port, or a universal serial bus. Wireless communication between input devices and the input/output interface 226 is possible as well, and includes infrared, BLUETOOTH^{®} wireless technology, IEEE 802.11a/b/g/n, cellular, ultra-wideband (UWB), ZigBee, LoRa, or other radio frequency communication systems in some possible embodiments.

In this example embodiment, a display device 228, such as a monitor, liquid crystal display device, projector, or touch sensitive display device, is also connected to the system bus 196 via an interface, such as a video adapter 230. In addition to the display device 228, the computing device 190 can include various other peripheral devices (not shown), such as speakers or a printer.

When used in a local area networking environment or a wide area networking environment (such as the Internet), the computing device 190 is typically connected to the network through a network interface 232, such as an Ethernet interface. Other possible embodiments use other communication devices. For example, some embodiments of the computing device 190 include a modem for communicating across the network.

The computing device 190 typically includes at least some form of computer readable media. Computer readable media includes any available media that can be accessed by the computing device 190. By way of example, computer readable media include computer readable storage media and computer readable communication media.

Computer readable storage media includes volatile and nonvolatile, removable and non-removable media implemented in any device configured to store information such as computer readable instructions, data structures, program modules or other data. Computer readable storage media includes, but is not limited to, random access memory, read only memory, electrically erasable programmable read only memory, flash memory or other memory technology, compact disc read only memory, digital versatile disks or other optical storage, magnetic cassettes, magnetic tape, magnetic disk storage or other magnetic storage devices, or any other medium that can be used to store the desired information and that can be accessed by the computing device 190.

Computer readable communication media typically embodies computer readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. By way of example, computer readable communication media includes wired media such as a wired network or direct-wired connection, and wireless media such as acoustic, radio frequency, infrared, and other wireless media. Combinations of any of the above are also included within the scope of computer readable media.

The computing device illustrated in FIG. 4 is also an example of programmable electronics, which may include one or more such computing devices, and when multiple computing devices are included, such computing devices can be coupled together with a suitable data communication network so as to collectively perform the various functions, methods, or operations disclosed herein.

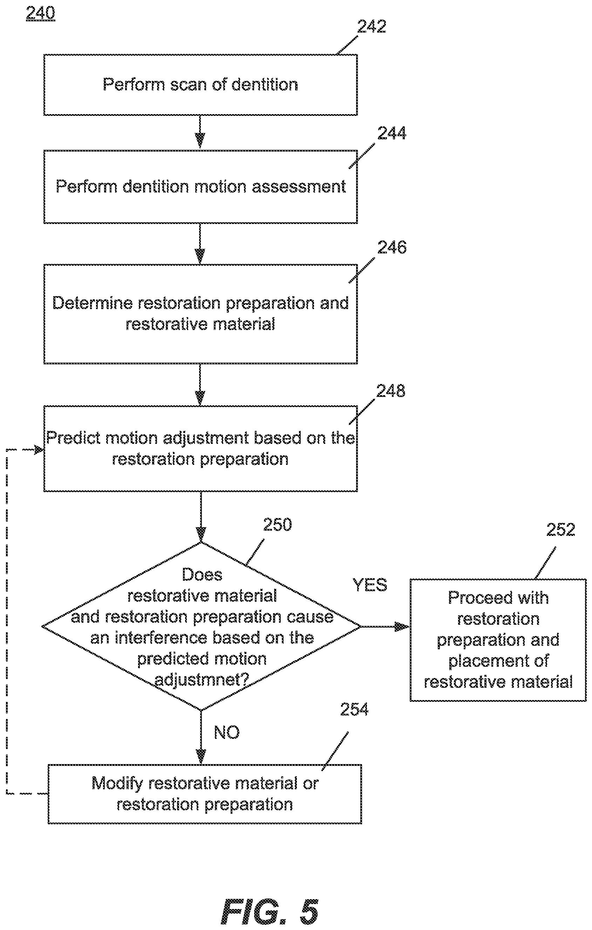

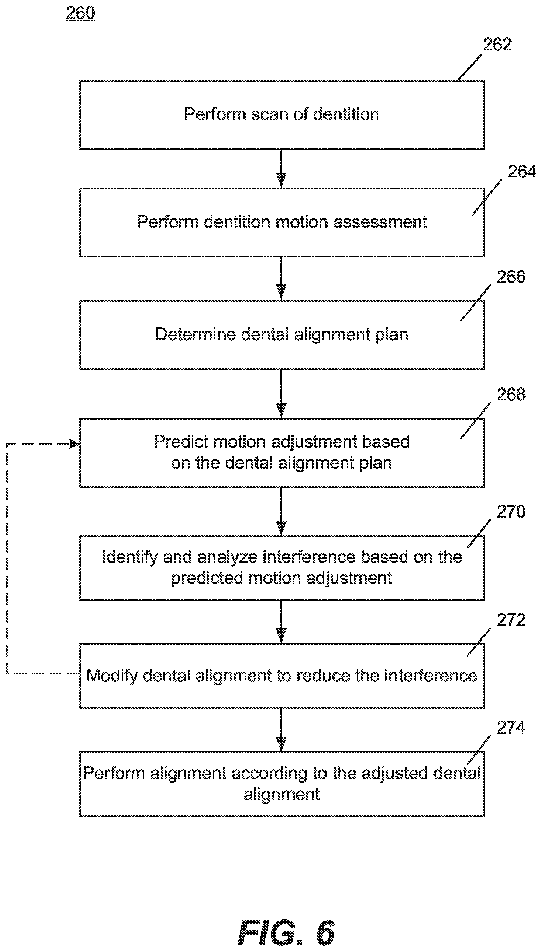

FIG. 5 is a flow chart illustrating an example method 240 of predicting a motion adjustment responsive to a restoration preparation. In this example, the method 240 includes operations 242, 244, 246, 248, 250, 252, and 254. Example dental procedures involving dentition adjustments may include dental restorations and dental alignment. The first example is illustrated and described in more detail with reference to FIG. 5, and the second example is illustrated and described in more detail with reference to FIG. 6.

Often, dental restorations involve two steps: preparation of the tooth or teeth for placement of restorative materials (i.e., a restoration preparation) and the placement of the restorative materials. Example dental restorations include dental implants, bridges, and/or crowns, among other similar dental procedures. The example method 240 can be implemented by various components of the patient evaluation system 120 described above with reference to FIGS. 2 and 3.

At operation 242, a scan 124 of dentition is performed prior to the preparation and/or placement of the restorative materials. Based on the scan 124, a position and orientation of each tooth in the upper and lower dental arches of the dentition is determined with respect to each other tooth. At operation 244, a dentition motion assessment 128 is performed. The dentition motion assessment 128 captures a representation of the movement of the dental arches relative to each other. For example, the dentition motion assessment 128 captures how each tooth in the upper and lower dental arches move with respect to one another.

An interference boundary prior to the restoration preparation (e.g., a pre-treatment interference boundary 154) can be determined from the scan 124 and the dentition motion assessment 128. The pre-treatment interference boundary 154 is a boundary of function between teeth of the dental arch on the upper jaw and teeth of the opposing dental arch on the lower jaw, and is defined by the interface or interaction between teeth on opposing dental arches. For example, the pre-treatment interference boundary 154 indicates how far each particular tooth can move vertically, anteroposteriorly, and transversely. If a tooth extends or crosses over the pre-treatment interference boundary in one or more planes, an interference with teeth of the opposing dental arch of the dentition is created.

At operation 246, a restorative material and a restoration preparation are determined. The restoration preparation can involve a removal and/or shaping of one or more portions of a tooth or teeth. In one example, the restoration preparation is a crown preparation that involves grinding off a top part of the tooth to replace with a crown. In another example, the restoration preparation is a bridge preparation that involves grinding off a top part of multiple teeth to replace with a bridge. In a further example, the restoration preparation is a dental implant preparation that involves removal of the entire tooth to be replaced with an implant.

In some examples, the restoration preparation is determined using the scan 124, data from the dentition motion assessment 128, and the determined pre-treatment interference boundary 154. For example, a position and orientation of a tooth associated with the restoration is visualized using the scan 124 and motion data 146 to determine how the tooth associated with the restoration preparation can move vertically, anteroposteriorly, and transversely ensure that the how the tooth is prepared (e.g., what portions of the tooth are being removed or how the tooth is shaped) does not move or adjust the tooth beyond the confines of or violate the pre-treatment interference boundary 154 to avoid introducing an interference.

The determination of the restorative material includes determining a type of material to use to replace or cover the one or more portions of the tooth or teeth removed and/or shaped in the restoration preparation. Based on a type of the restoration preparation, certain types of restorative material can be more advantageous to use due to the properties of the materials, such as a durability of the material or a malleability of the material. A size of the material, including a thickness of material, can also be determined.

At operation 248, a motion adjustment based on the restoration preparation is predicted. As one example, if the restoration preparation determined at operation 246 is a crown preparation that will require the cutting or grinding down of a tooth or set of teeth, the additional space provided by the crown preparation may actually modify the patient's bite and jaw motion by removing an interference that had been previously restricting the motion of the jaw. This modification to the bite and jaw motion may result in the need to further modify the restoration preparation and/or restorative materials determined at operation 246 to avoid interferences resulting from the modifications. For example, the jaw can now move more freely in one or more directions causing the teeth to move relative to one another in different ways (e.g., a particular tooth can now move further vertically, anteroposteriorly, or transversely). Accordingly, the motion adjustment can affect the pre-treatment interference boundary 154.

Therefore, to predict the motion adjustment, a determination is made as to whether an interference will be removed after the restoration preparation, and if so, associated effects of the removal by predicting a post-preparation interference boundary. The post-preparation interference boundary is defined by the interface or interaction between the teeth on the opposing dental arches after the restoration preparation is performed. In some examples, the post-preparation interference boundary can be compared to the pre-treatment interference boundary 154 to measure a change that has occurred, where the change (e.g., a net reduction) between the two interference boundaries can be used to determine whether the dentition has been sufficiently prepared for the placement of the restorative material. Continuing the above example of a crown preparation, a particular amount of reduction (e.g., cutting or grinding down of the tooth) is required so that a restorative material of a sufficient thickness (e.g., for stability) can be placed over the prepared tooth without causing interference. In other words, the particular amount of reduction during preparation with placement of the sufficiently thick restorative material thereon meets a required clearance to avoid interference.

At decision 250, a determination is made as to whether the restorative material and the restoration preparation cause an interference based on the predicted motion adjustment. The determination can be made based on the measured change between the pre-treatment interference boundary 154 and the post-preparation interference boundary predicted discussed above in conjunction with operation 248. In certain embodiments, a post-preparation scan may be performed to confirm the predicted post-preparation interference boundary described above. If there are variations between the post-preparation scan and the predicted post-preparation interference boundary, necessary adjustments can be identified to allow the dentist to adjust the preparation to more closely match the predicted preparation.

As previously discussed, dental restoration involves two steps: preparation of the tooth for placement of restorative materials (i.e., restoration preparation) and the placement of the restorative materials. Restoration preparation includes a change to a structure or shape of a tooth or teeth. For example, grinding down a tooth to a certain size and shape, so that it may be covered with restorative material (e.g. a crown) or extracting one or more teeth for installation of a dental implant or a bridge. As described above with respect to operation 246, the restoration preparation and restorative materials are determined based on the pre-treatment interference boundary 154 to avoid introducing interference. For example, the restoration preparation and restorative materials are initially determined to meet a required clearance, which ensures a proper fit enabling the patient P to maintain a same or similar bite pre-treatment and post-dentition adjustment (e.g., a tooth and restorative material when placed over the tooth does not extend or cross over the pre-treatment interference boundary 154 in one or more planes to avoid introducing interference). However, any adjustments to the motion of the jaw resulting from the restoration preparation of the tooth (e.g., due to a removal of an interference) can correspondingly impact how the tooth should be prepared and/or how the restorative materials should be placed. For example, if an interference is removed by the restoration preparation and the motion of the jaw changes, the required clearance could either increase or decrease in size due to a change in the pre-treatment interference boundary 154, and the restoration preparation and restorative materials may no longer avoid interference. As one example, when the changes in jaw motion cause the required clearance to decrease in size (e.g., when a space in which the restorative material placed over the prepared tooth is designed to fit has decreased), a vertical height of the restorative material, such as a crown, may now be too tall for the space causing a new interference with opposing teeth.

If the restorative material and the restoration preparation do not cause an interference based on the predicted motion adjustment (e.g., based on the measured change between the pre-treatment interference boundary 154 and the post-preparation interference boundary), the restoration preparation is performed and the restorative material is placed at operation 252. For example, the portions of the tooth are removed and/or shaped according to the initial restoration preparation determined, and the portions removed can be replaced with the initial restorative materials determined.

If the restorative material and the restoration preparation cause an interference based on the predicted motion adjustment (e.g., based on the measured change between the pre-treatment interference boundary 154 and the post-preparation interference boundary), one or more of the restorative material and the restoration preparation are modified at operation 254. For example, the restoration preparation and/or restorative material are modified to correspond to the predicted post-preparation interference boundary. In one embodiment, a size or a thickness of the restorative material can be modified or a different type of restorative material can be used. For example, if it is predicted that the restoration preparation will remove an interference and cause a motion adjustment of the jaw that lowers a height of clearance for the restorative materials being placed (e.g., the motion adjustment brings upper and lower dentition closer together leaving less space for the restorative material), a size or thickness of the restorative material is reduced. However, in some cases, properties of the restorative material cannot be reduced below a certain size or thickness and thus a new restorative material will be selected. In another embodiment, the restoration preparation itself is adjusted. For example, a tooth being prepared can be ground or cut down to a different height or shape that will enable the restorative material to be placed. In a further embodiment, both the restorative material and the restoration preparation are modified.

In additional embodiments, when the restoration preparation includes a crown preparation and the restorative material is a crown, the crown can be automatically generated to account for the motion adjustment. For example, an occlusal surface of the crown may be generated digitally to correspond with the predicted post-preparation interference boundary. In some embodiments, a default crown structure is used as a starting template that is then adjusted and contoured based on the predicted post-preparation interference boundary. Specifically, the predicted post-preparation interference boundary is used to define contoured portions of the crown surface that interface with opposing and adjacent teeth to prevent introducing interference. For example, based on the predicted post-preparation interference boundary, one or more rules or parameters are generated to ensure that the boundary is not violated in any plane by the crown as it is being digitally generated. In some examples, the rules or parameters may be associated with a slope of the sidewalls of the crown, in addition to a height and shape of the crown.

In some examples, crown preparation reduction may be evaluated virtually prior to application of a crown. In such examples, an additional scan of the patient's dentition would be made after reduction but before the crown is applied. Including a scan of the reduction would allow the dentist to inspect the reduction digitally from any desired angle to confirm that the correct amount of reduction has been performed. A similar process could be used during preparation for either full or partial dentures. For implant supported dentures, the reduction surface would be gum tissue or implants upon which dentures would sit, but the concept is the same. Scanning the prepared gum tissue or implants would allow a dentist to inspect the preparations virtually to confirm that the denture design would establish a vertical dimension (height) for the teeth that would avoid any undesirable interference and provide a sufficient gap (freeway space) between the upper and lower teeth when the patient is at rest. For conventional dentures, scanning soft tissue would allow a dentist to virtually simulate the try-in process, thereby providing guidance regarding whether material should be added or removed from bite rims to, again, avoid interference and provide a sufficient gap between the upper and lower teeth when the patient is at rest. In certain embodiments, custom bite rims could be printed based on the simulation.

In some embodiments, the operations 248, 250, and 254 can be repeated one or more times until the restorative material and the restoration preparation do not cause an interference. The process can be iteratively repeated to modify the restorative material or restoration preparation, predict motion based on the modified restoration preparation, and further modify the restorative material or restoration reparation based on whether the restorative material and restoration preparation cause an interference based on the predicted motion.

FIG. 6 is a flow chart illustrating an example method 260 of predicting a motion adjustment responsive to a dental alignment plan. The dental alignment plan involves adjustment of position and/or alignment of the teeth in three planes of space, which can be accomplished using braces, retainers, or other dental alignment tools. The example method 260 can be implemented by various components of the patient evaluation system 120 described above in FIGS. 2 and 3. In this example, the method 260 includes operations 262, 264, 266, 268, 270, 272, and 274.

At operation 262, a scan 124 of dentition is performed. Based on the scan 124, a position and orientation of each tooth in the upper and lower dental arches is determined with respect to each other tooth. At operation 264, a dentition motion assessment 128 is performed. The dentition motion assessment 128 captures a representation of the movement of the dental arches relative to each other. For example, the dentition motion assessment 128 captures how each tooth in the upper and lower dental arches move with respect to one another. The movement is captured as motion data 146.

Using the scan 124 and motion data 146, an interference boundary prior to the dental alignment (e.g., a pre-treatment interference boundary 154) can be determined. The pre-treatment interference boundary 154 is a boundary of function between teeth of the dental arch on the upper jaw and teeth of the opposing dental arch on the lower jaw, and is defined by the interface or interaction between teeth on opposing dental arches. For example, the pre-treatment interference boundary 154 indicates how far a particular tooth can move vertically, anteroposteriorly, and transversely. If a tooth extends or crosses over the pre-treatment interference boundary 154 in one or more planes, an interference with teeth of the opposing dental arch of the dentition is created.

At operation 266, a dental alignment plan is determined using the scan 124 of the dentition, motion data 146 from the dentition motion assessment 128, and the pre-treatment interference boundary 154. For example, utilizing the scan 124 and motion data 146, a position and orientation of a tooth associated with the alignment and how the tooth can move vertically, anteroposteriorly, and transversely can be visualized to ensure that how the tooth is aligned (e.g., a direction or orientation of the alignment) does not move or adjust the tooth beyond the confines of the pre-treatment interference boundary 154 to avoid introducing an interference.

At operation 268, a motion adjustment based on the dental alignment plan is predicted. As one example, if the dental alignment plan determined at operation 266 involves straightening a crooked tooth, the new position and orientation of the straight tooth can cause the patient's bite and jaw motion to change. This change to the bite and jaw motion may result in the need to further modify the dental alignment plan determined at operation 246 to avoid interferences resulting from the changes. For example, the motion of the jaw may be more restricted in one or more directions causing the teeth to move relative to one another in different ways. For example, the tooth once straightened can no longer move as far vertically, anteroposteriorly, or transversely before interfacing with opposing teeth. Accordingly, the motion adjustment can affect the pre-treatment interference boundary 154.

Therefore, to predict the motion adjustment, a determination is made as to whether an interference will be removed or created following the dental alignment and if so, associated effects of the removal by predicting a post-alignment interference boundary (e.g., the interference boundary after the dental alignment). The post-alignment interference boundary is defined by the interface or interaction between the teeth on the opposing dental arches after the dental alignment.

At operation 270, an interference is identified and analyzed based on the predicted motion adjustment. For example, in some embodiments, the interference is identified and analyzed by comparing the pre-treatment interference boundary 154 to the post-alignment interference boundary.

At operation 272, the dental alignment is modified to reduce the interference. For example, the dental alignment plan is modified and the modified dental alignment can be further analyzed to see if the interference has been removed. If not, the plan can be iteratively modified until the interference has been eliminated or is less than a predetermined threshold amount. For example, the operations 268, 270, and 272 can be repeated one or more times to adjust the dental alignment, predict motion based on the adjusted dental alignment, identify and analyze interference based on the predicted motion, and further adjust the dental alignment based on identified interference until interference is eliminated, or until the interference is less than a predetermined amount. Once the interference is eliminated, or the interference is less than the predetermined amount, the dental alignment can be performed according to the adjusted dental alignment at operation 274. The iterations described above can be performed at the outset of treatment or, in some cases, may be performed at certain milestones during the course of treatment. In such cases, additional scans would be taken throughout the treatment process to confirm the dental alignment plan or, if the actual motion varies from the predicted motion, the alignment plan can be modified based on an updated motion prediction.

In certain embodiments, it may be desirable to develop a dental treatment plan with comparable accuracy to the methods described above but without needing to obtain a scan 124 of a patient's dentition using scanner 122 or motion capture station 126, which tend to be expensive and may be time consuming. This becomes possible as the volume of patient scan data increases over time.

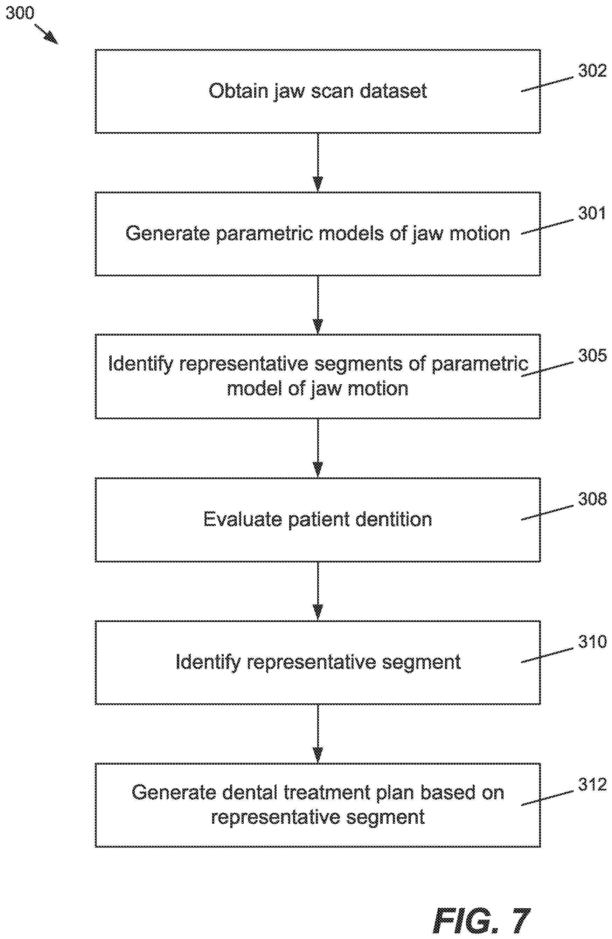

In the method 300 shown in FIG. 7, scan data of jaw size, dentition motion and interference boundaries generated therefrom may be obtained from any suitable source and stored in a dataset 302. In certain embodiments, dataset 302 may be stored locally on the computing device 190, but may be periodically updated by installing an updated dataset 302 that is downloaded from a vendor website, stored on a portable storage device, or by any other suitable means. In certain embodiments, dataset 302 may be provided by a vendor that obtains jaw data from any number of sources. In other words, although it could be generated locally, the data contained in dataset 302 can be obtained from any suitable source.

At operation 301, the dataset 302 is analyzed to generate a parametric model of human jaw motion 304. Next, at operation 305, the parametric model of jaw motion 304 is divided into representative segments 306 that may be segmented by any desired parameter including, but not limited to size and/or jaw motion type. In certain embodiments, the parameters may include information such as lip profile, tooth length, malocclusion, or even the age of the subject. As with any parametric model, as the amount of data (sample size) increases, the accuracy of the model increases as well. Inclusion of a subject's age may provide useful because human jaw motion changes as we age. The parametric model of jaw motion 304 represents a true anatomic motion database that, when the data is analyzed together, can be used to predict and validate motion of a patient's jaw that is not included in the dataset 302. In the non-limiting example shown, representative segments 306 include parameters included in a patient jaw profile 320.

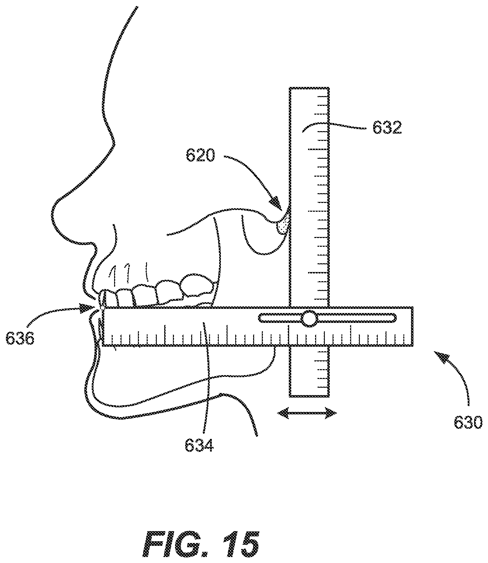

As shown in FIGS. 12-15, patient jaw profile 320 may be measured in a number of suitable ways including, but not limited to, measuring the total length of the lower jaw 608 by adding the distance between the midpoint of the tragus and soft tissue around the angle 609 of the mandible to the distance between the angle and the soft tissue in the region of the chin. It is also desirable to measure mandibular width 610, which is the distance between the two angles 609 of the lower jaw 608. The anterior-posterior distance of the arch from the midline to the retromolar pad may also be measured, along with other measurements related to tooth location and bite profile may be included. None of these measurements require the use of scanner 122 or motion capture station 126. Rather, the measurements can be taken manually using standard tools present in most dental offices. One example measuring tool 630 is shown in FIG. 15, and includes two rulers 632, 634 slidably attached to each other and disposed at a 90-degree angle. Measuring tool 630 is used to measure a horizontal distance from an incisal reference point 636 to the approximate position of the condyle 620 when the jaw 602 is closed as well as the vertical distance from the condyle to the incisal reference point. As shown, the incisal reference point 636 is the bite surface of a lower incisor. In some embodiments, the measurements may also be taken from radiographs or any other suitable means. Of course, determining which measurements of patient jaw profile 320 to take depends on the parameters used in the parametric model of jaw motion 304.

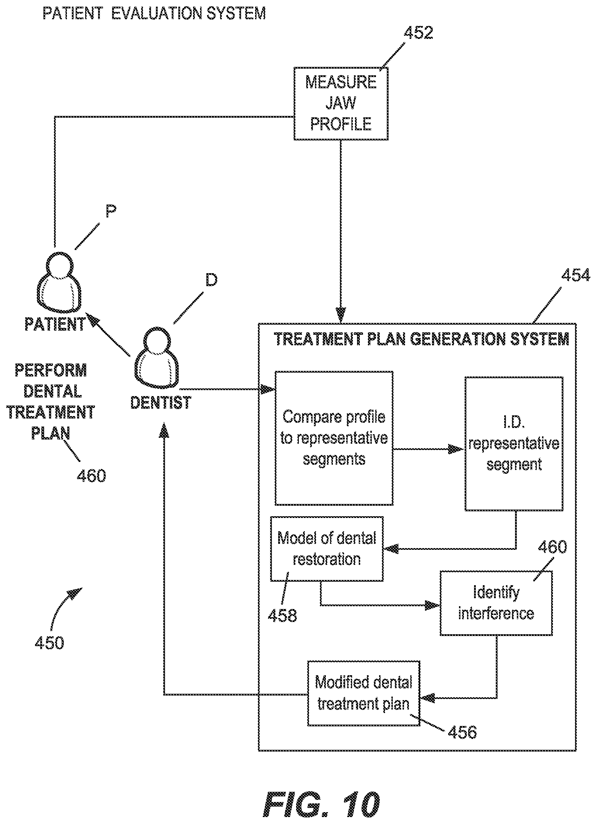

Returning to the method 300 shown in FIG. 7, operation 308 includes the evaluation of patient dentition, which in the embodiment shown is the identification of the patient jaw profile 320. Next, at operation 310, the patient jaw profile 320 is compared to the representative segments 306 in operation 310 to identify the representative segment 306 that most closely matches the patient jaw profile. In certain embodiments, the parametric model correlates true anatomic motion data included in dataset 302 and with static measurements to identify the appropriate representative segment 306. Finally, a dental treatment plan 456 (see FIG. 10) is generated based on the representative segment 306 identified during operation 310.

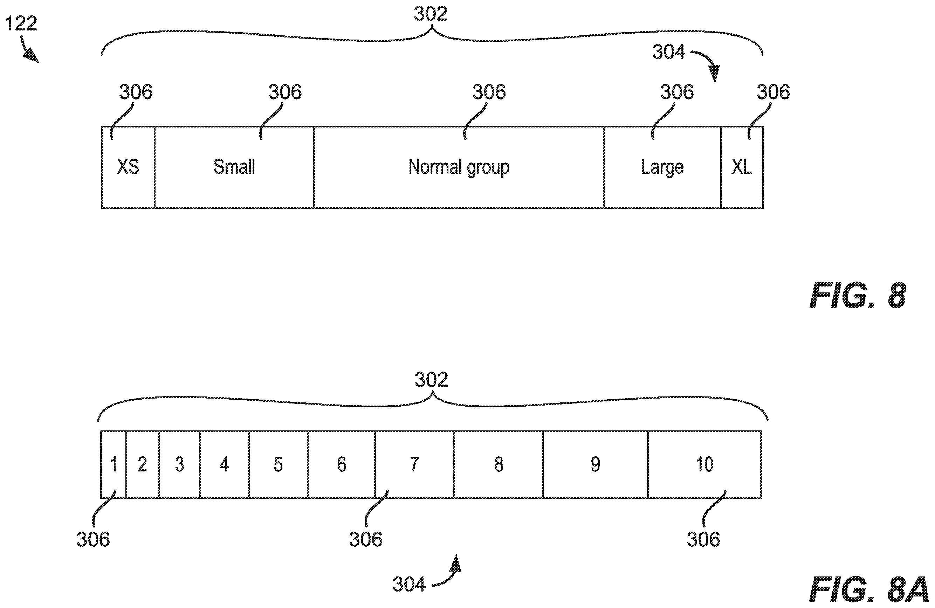

A non-limiting example of dataset 302 is shown in FIG. 8. As shown, dataset 302 is divided into five representative segments 306 (Extra Small, Small, Normal, Large, and Extra Large). The representative segments 306 correspond to a normal probability distribution (bell curve), wherein the majority of cases would fall into the "Normal" category with "Small" and "Large" being one standard deviation smaller and larger, respectively, and "Extra Small" and "Extra Large" being two standard deviations smaller and larger than "Normal."

In certain embodiments it may be desirable to subdivide the representative segments 306, which may further improve the accuracy of the treatment plan generated by the method described in detail below. Of course, dataset 302 could be divided into as many categories as desired without departing from the scope of the appended claims. Another example of dataset 302 is shown in FIG. 8A. In FIG. 8A, dataset 302 is divided into ten sizes (1-10).

As more and more scan data is entered into dataset 302, the accuracy of representative segments 306 improves and it becomes possible to estimate, with accuracy, a patient's jaw motion by determining which of the representative segments 306 most closely matches the patient jaw profile 320. In certain embodiments, the representative segment 306 comprises data from an individual scan contained in the dataset 302. In certain other embodiments, representative segments 306 may comprise an aggregation of data that falls within a range of parameters, to create an "ideal" representative jaw profile. Once the appropriate representative segment 306 is identified, the representative segment data is used to in place of scan 124 and dentition motion assessment 128 used in the system and method described previously, i.e., it is used to generate a dental treatment plan 308. In this way, it is possible to obtain a dental treatment plan 308 that approximates of the benefits of using scan 124 and dentition motion assessment 128 by taking only some basic jaw measurements. In some embodiments, additional measurements or assessments may be obtained by a dentist related to the patient jaw profile 320 without departing from the scope of the appended claims. Indeed, in some cases taking additional measurements may result in a more accurate identification of the appropriate representative segment 306. For example, if certain asymmetries are measured on the patient, that information may be used to identify a representative segment 306 having similar asymmetries.

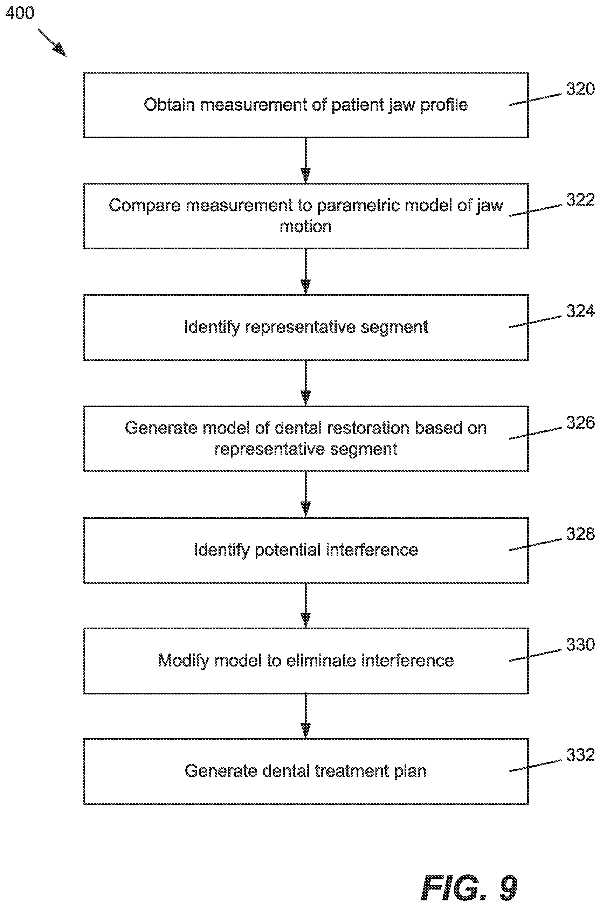

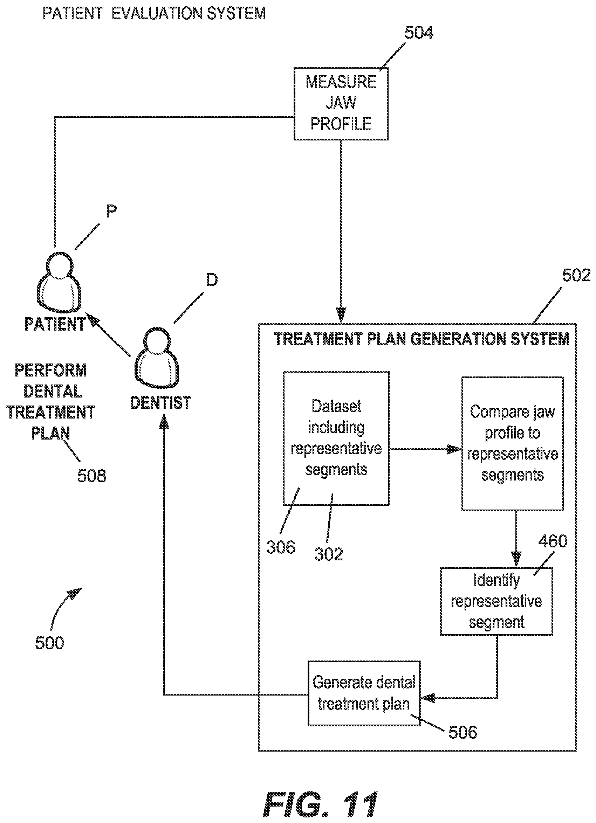

FIG. 9 is a flow chart showing another method 400 of developing a dental treatment plan using the patient jaw profile 320. In operation 320, a dentist obtains the patient jaw profile 320 by taking pertinent measurements of the patient's jaw as described above. Next, in operation 322, the dentist inputs the patient jaw profile into treatment plan generation system 454 (see FIG. 11), which compares the patient jaw profile 320 measurement to the parametric model of jaw motion 304. Next, in operation 324, a representative segment 306 is identified that most closely matches the patient jaw profile 320. In operation 326, the representative segment 306 identified in operation 324 is used to generate a model of dental restoration 458 (see FIG. 11). Once the model of dental restoration 458 is generated, treatment plan generation system 454 (see FIG. 11) identifies potential interference in operation 328. Treatment plan generation system 434 modifies the model of dental restoration 458 in optional operation 330 to eliminate any identified interferences. Finally, in operation 332, treatment plan generation system 454 generates a final dental treatment plan 456 that is used by the dentist to treat the patient.

FIG. 11 is a schematic block diagram illustrating an example patient evaluation system 450 for implementing the method 400 of analyzing a dental treatment plan described in FIG. 9. As shown, system 450 includes treatment plan generation system 454. Prior to a dentist D performing a dental procedure on a patient P, a measurement 452 of the dentition of the patient P is performed to obtain the patient jaw profile 320. The patient jaw profile 320 is provided as input to the treatment plan generation system 454.

Based on the patient jaw profile 320, the treatment plan generation system 454 determines a dental treatment plan 456. The dental treatment plan 456 can involve any kind of change in the structure or positions of the teeth, such as a removal, re-shaping, and/or re-alignment of a portion or entirety of a tooth or teeth on one or both dental arches of the patient P's dentition, among other examples. When determining the dental treatment plan 456, the patient jaw profile 320 is compared to the representative segments 306 from dataset 302. Treatment plan generation system 454 identifies the appropriate representative segment 306 that is most similar to the patient jaw profile 320. Next, the system 454 creates a model of dental restoration 458 based on the representative segment 306 and the treatment to be performed. The model of dental restoration 458 may include interferences 460, which the system 454 identifies before generating a modified dental treatment plan 456 that avoids any such interferences. By generating dental treatment plan 456 based on the known data contained in representative segments 306, the treatment plan reduces the likelihood of significant interferences, which also reduces the frequency and severity of necessary adjustments during the course of treatment.

Because changes can occur in the patient's jaw motion during the course of treatment, the treatment plan generation system 454 can be used in subsequent appointments to determine whether the representative segment 306 used to develop the original dental treatment plan 456 is still the correct representative segment. If not, adjustments to the dental treatment plan 456 can be made based on a new representative segment 306, as needed. In certain embodiments, representative segments 306 may be configured to avoid any interferences, regardless of the treatment being done, so no adjustment to avoid such interferences is made.

The treatment plan generation system 454 uses predicted motion adjustment to modify the model of dental restoration 458 to create dental treatment plan 456. The treatment plan generation system 454 provides the dental treatment plan 456 as output to the dentist D such that the dentist D performs 460 the dental treatment plan that proactively avoids introducing interferences.

Turning now to FIG. 12, another schematic block diagram illustrating an example patient evaluation system 500 is shown. Patient evaluation system 500 corresponds to the method 300 of FIG. 7. The patient evaluation system 500 includes a treatment plan generation system 502. Prior to a dentist D performing a dental procedure on a patient P, a measurement 504 of the dentition of the patient P is performed to obtain the patient jaw profile 320. The patient jaw profile 320 is provided as input to the treatment plan generation system 502.

Based on the patient jaw profile 320, the treatment plan generation system 502 determines a dental treatment plan 506. As in the previously described embodiments, the dental treatment plan 506 can involve any kind of change in the structure or positions of the teeth, such as a removal, re-shaping, and/or re-alignment of a portion or entirety of a tooth or teeth on one or both dental arches of the patient P's dentition, among other examples. When determining the dental treatment plan 506, the patient jaw profile 320 is compared to the representative segments 306 from dataset 302. Treatment plan generation system 502 identifies the appropriate representative segment 306 that is most similar to the patient jaw profile 320. Next, the system 502 generates the dental treatment plan 506. Generating dental treatment plan 506 based on the known data contained in representative segments 306 reduces the likelihood of significant interferences, which also reduces the frequency and severity of necessary adjustments during the course of treatment.

Because changes can occur in the patient's jaw motion during the course of treatment, the treatment plan generation system 502 can be used in subsequent appointments to determine whether the representative segment 306 used to develop the original dental treatment plan 506 is still the correct representative segment. For example, mid-treatment measurements may be taken and put through treatment plan generation system 502 to confirm the original representative segment 306. Treatment plan generation system 502 may, however, determine based on the mid-treatment measurements that a different representative segment 306 would be more appropriate. In that case, adjustments to the dental treatment plan 506 created at the beginning of treatment can be modified based on the newly identified representative segment 306. In this embodiment, representative segments 306 may be configured to avoid any interferences, regardless of the treatment being done, so no adjustment to avoid such interferences is made.

The treatment plan generation system 502 uses predicted motion adjustment to create dental treatment plan 506. The treatment plan generation system 502 provides the dental treatment plan 506 as output to the dentist D such that the dentist D performs 508 the dental treatment plan that proactively avoids introducing interferences.

The various embodiments described above are provided by way of illustration only and should not be construed to limit the claims attached hereto.

## Claims

1. A computer-implemented method of generating a dental treatment plan comprising the steps of:
obtaining a dataset of human jaw and dentition motion and measurements;
generating a parametric model of jaw motion based on the dataset of human jaw and dentition motion and measurement;
identifying a plurality of representative segments of the parametric model of jaw motion;
evaluating a patient dentition to generate a patient jaw profile, the evaluation including measuring at least one parameter of the patient's jaw;
identifying one of the representative segments of the parametric model of jaw motion that is most similar to the patient jaw profile; and
generating the dental treatment plan based on the representative segment of the parametric model of jaw motion.

2. The computer-implemented method of claim 1, further comprising the step of measuring the size of the patient's jaw.

3. The computer-implemented method of claim 1, wherein the representative segments are segmented by jaw size,

4. The computer-implemented method of claim 1, wherein the representative segments are segmented by jaw motion type.

5. The computer-implemented method of claim 1, wherein the parametric model of jaw motion is divided into five representative segments corresponding with sizes extra small, small, normal, large, and extra large.

6. The computer-implemented method of claim 1, wherein the representative segments are determined using a normal probability distribution curve, wherein segments are determined by standard deviations from the mean.

7. The computer-implemented method of claim 1, wherein the representative segments comprise three-dimensional digital dental models generated from scans and a pre-treatment interference boundary determined from motion data included in the dataset of human jaw and dentition motion and measurements.

8. The computer-implemented method of claim 1, further including the step of predicting a post-treatment interference boundary based on the representative segment identified as most similar to the patient jaw profile.

9. The computer-implemented method of claim 8, wherein predicting the post-treatment interference boundary comprises:
predicting the post-treatment interference boundary manually using a digital articulator displayed through a user interface of a computing device, the digital articulator including a three-dimensional (3-D) digital dental model generated from the representative segment and simulated to include the dentition adjustment, wherein a user interacts with the digital articulator using input devices of the computing device to manually shift a lower jaw within the simulation.

10. A data processing system comprising means for generating a dental treatment plan comprising:
a treatment plan generator configured to determine a treatment plan based on a patient jaw profile;
the patient jaw profile including at least one measurement of a patient's jaw size;
the treatment plan generator including a parametric model of jaw motion based on a dataset of human jaw and dentition motion and measurement;
the parametric model of jaw motion divided into a plurality of representative segments; and
the treatment plan generator further configured to compare the patient jaw profile to the parametric model of jaw motion to determine which of the representative segments is most similar to the patient jaw profile and to generate the dental treatment plan based on the representative segment identified as most similar to the patient jaw profile.

11. The system of claim 10, wherein the treatment plan generator is further configured to:
generate a model of dental restoration based on the representative segment that is most similar to the patient jaw profile;
identify interferences; and
generate a modified dental treatment plan that avoids the identified interferences.

12. The system of claim 10, wherein the dataset of human jaw and dentition motion and measurement includes three-dimensional digital dental models generated from scans and a pre-treatment interference boundary determined from motion data.

13. The system of claim 10, further comprising an interference boundary generator, wherein the interference boundary generator is configured to:
determine a pre-treatment interference boundary based on the representative segment identified as most similar to the patient jaw profile; and
provide the pre-treatment interference boundary to the dentition treatment plan generator.

14. The system of claim 13, wherein the treatment plan generator is configured to further determine the treatment plan based on the pre-treatment interference boundary.

## Patentansprüche

1. Ein computerimplementiertes Verfahren zum Erzeugen eines dentalen Behandlungsplans, umfassend die Schritte:
Erhalten eines Datensatzes von Bewegung und Messungen eines menschlichen Kiefers und eines Gebisses;
Erzeugen eines parametrischen Modells einer Kieferbewegung basierend auf dem Datensatz von Bewegung und Messungen des menschlichen Kiefers und Gebisses;
Identifizieren einer Mehrzahl repräsentativer Segmente des parametrischen Modells der Kieferbewegung;
Bewerten eines Patientengebisses, um ein Patientenkieferprofil zu erzeugen, wobei das Bewerten ein Messen wenigstens eines Parameters des Kiefers des Patienten umfasst;
Identifizieren eines der repräsentativen Segmente des parametrischen Modells der Kieferbewegung, das dem Patientenkieferprofil am ähnlichsten ist; und
Erzeugen des dentalen Behandlungsplans basierend auf dem repräsentativen Segment des parametrischen Modells der Kieferbewegung.

2. Das computerimplementierte Verfahren nach Anspruch 1, ferner umfassend den Schritt des Messens der Größe des Kiefers des Patienten.

3. Das computerimplementierte Verfahren nach Anspruch 1, wobei die repräsentativen Segmente nach Kiefergröße segmentiert sind.

4. Das computerimplementierte Verfahren nach Anspruch 1, wobei die repräsentativen Segmente nach Kieferbewegungstyp segmentiert sind.

5. Das computerimplementierte Verfahren nach Anspruch 1, wobei das parametrische Modell der Kieferbewegung in fünf repräsentative Segmente unterteilt ist, die den Größen extra klein, klein, normal, groß und extra groß entsprechen.

6. Das computerimplementierte Verfahren nach Anspruch 1, wobei die repräsentativen Segmente unter Verwendung einer Normalwahrscheinlichkeitsverteilungskurve bestimmt werden, wobei Segmente durch Standardabweichungen vom Mittelwert bestimmt werden.

7. Das computerimplementierte Verfahren nach Anspruch 1, wobei die repräsentativen Segmente dreidimensionale digitale Dentalmodelle umfassen, die aus Scans erzeugt wurden, und eine Vorbehandlungs-Interferenzgrenze, die aus Bewegungsdaten bestimmt wurde, die in dem Datensatz von Bewegung und Messungen des menschlichen Kiefers und Gebisses enthalten sind.

8. Das computerimplementierte Verfahren nach Anspruch 1, ferner umfassend den Schritt des Vorhersagens einer Nachbehandlungs-Interferenzgrenze basierend auf dem repräsentativen Segment, das als dem Patientenkieferprofil am ähnlichsten identifiziert wurde.

9. Das computerimplementierte Verfahren nach Anspruch 8, wobei das Vorhersagen der Nachbehandlungs-Interferenzgrenze umfasst:
manuelles Vorhersagen der Nachbehandlungs-Interferenzgrenze unter Verwendung eines digitalen Artikulators, der über eine Benutzerschnittstelle einer Rechenvorrichtung angezeigt wird, wobei der digitale Artikulator ein dreidimensionales (3-D) digitales Dentalmodell umfasst, das aus dem repräsentativen Segment erzeugt und simuliert wurde, um die Gebissanpassung zu umfassen, wobei ein Benutzer mit dem digitalen Artikulator unter Verwendung von Eingabevorrichtungen der Rechenvorrichtung interagiert, um einen Unterkiefer innerhalb der Simulation manuell zu verschieben.

10. Ein Datenverarbeitungssystem, umfassend Mittel zum Erzeugen eines dentalen Behandlungsplans, wobei die Mittel umfassen:
einen Behandlungsplan-Generator, der dazu eingerichtet ist, einen Behandlungsplan basierend auf einem Patientenkieferprofil zu bestimmen;
wobei das Patientenkieferprofil wenigstens eine Messung einer Kiefergröße eines Patienten umfasst;
wobei der Behandlungsplan-Generator ein parametrisches Modell einer Kieferbewegung basierend auf einem Datensatz von Bewegung und Messungen des menschlichen Kiefers und Gebisses umfasst;
wobei das parametrische Modell der Kieferbewegung in eine Mehrzahl repräsentativer Segmente unterteilt ist; und
wobei der Behandlungsplan-Generator ferner dazu eingerichtet ist, das Patientenkieferprofil mit dem parametrischen Modell der Kieferbewegung zu vergleichen, um zu bestimmen, welches der repräsentativen Segmente dem Patientenkieferprofil am ähnlichsten ist, und den dentalen Behandlungsplan basierend auf dem repräsentativen Segment zu erzeugen, das als dem Patientenkieferprofil am ähnlichsten identifiziert wurde.

11. Das System nach Anspruch 10, wobei der Behandlungsplan-Generator ferner zu Folgendem eingerichtet ist:
Erzeugen eines Modells einer dentalen Restauration basierend auf dem repräsentativen Segment, das dem Patientenkieferprofil am ähnlichsten ist;
Identifizieren von Interferenzen; und
Erzeugen eines modifizierten dentalen Behandlungsplans, der die identifizierten Interferenzen vermeidet.

12. Das System nach Anspruch 10, wobei der Datensatz von Bewegung und Messungen des menschlichen Kiefers und Gebisses dreidimensionale digitale Dentalmodelle umfasst, die aus Scans erzeugt wurden, und eine Vorbehandlungs-Interferenzgrenze, die aus Bewegungsdaten bestimmt wurde.

13. Das System nach Anspruch 10, ferner umfassend einen Interferenzgrenzen-Generator, wobei der Interferenzgrenzen-Generator zu Folgendem eingerichtet ist:
Bestimmen einer Vorbehandlungs-Interferenzgrenze basierend auf dem repräsentativen Segment, das als dem Patientenkieferprofil am ähnlichsten identifiziert wurde; und
Bereitstellen der Vorbehandlungs-Interferenzgrenze an den Gebissbehandlungsplan-Generator.

14. Das System nach Anspruch 13, wobei der Behandlungsplan-Generator dazu eingerichtet ist, den Behandlungsplan ferner basierend auf der Vorbehandlungs-Interferenzgrenze zu bestimmen.

## Revendications

1. Procédé mis en œuvre par ordinateur de génération d'un plan de traitement dentaire comprenant les étapes de :
obtention d'un ensemble de données de mouvement et de mesures de mâchoire et de denture humaines ;
génération d'un modèle paramétrique de mouvement de mâchoire sur la base de l'ensemble de données de mouvement et de mesure de mâchoire et de denture humaines ;
identification d'une pluralité de segments représentatifs du modèle paramétrique de mouvement de mâchoire ;
évaluation d'une denture de patient pour générer un profil de mâchoire du patient, l'évaluation comportant la mesure d'au moins un paramètre de la mâchoire du patient ;
identification de l'un des segments représentatifs du modèle paramétrique de mouvement de mâchoire qui est le plus similaire au profil de mâchoire du patient ; et
génération du plan de traitement dentaire sur la base du segment représentatif du modèle paramétrique de mouvement de mâchoire.

2. Procédé mis en œuvre par ordinateur selon la revendication 1, comprenant en outre l'étape de mesure de la taille de la mâchoire du patient.

3. Procédé mis en œuvre par ordinateur selon la revendication 1, dans lequel les segments représentatifs sont segmentés par taille de mâchoire.

4. Procédé mis en œuvre par ordinateur selon la revendication 1, dans lequel les segments représentatifs sont segmentés par type de mouvement de mâchoire.

5. Procédé mis en œuvre par ordinateur selon la revendication 1, dans lequel le modèle paramétrique de mouvement de mâchoire est divisé en cinq segments représentatifs correspondant à des tailles très petite, petite, normale, grande et très grande.

6. Procédé mis en œuvre par ordinateur selon la revendication 1, dans lequel les segments représentatifs sont déterminés à l'aide d'une courbe de distribution de probabilité normale, dans lequel les segments sont déterminés par des écarts-types par rapport à la moyenne.

7. Procédé mis en œuvre par ordinateur selon la revendication 1, dans lequel les segments représentatifs comprennent des modèles dentaires numériques tridimensionnels générés à partir de balayages et d'une limite d'interférence de prétraitement déterminée à partir de données de mouvement incluses dans l'ensemble de données de mouvement et de mesures de mâchoire et denture humaines.

8. Procédé mis en œuvre par ordinateur selon la revendication 1, comportant en outre l'étape de prédiction d'une limite d'interférence de post-traitement sur la base du segment représentatif identifié comme le plus similaire au profil de mâchoire du patient.

9. Procédé mis en œuvre par ordinateur selon la revendication 8, dans lequel la prédiction de la limite d'interférence de post-traitement comprend :
la prédiction manuelle de la limite d'interférence de post-traitement à l'aide d'un articulateur numérique affiché par le biais d'une interface utilisateur d'un dispositif informatique, l'articulateur numérique comportant un modèle dentaire numérique tridimensionnel (3-D) généré à partir du segment représentatif et simulé pour inclure l'ajustement de la denture, dans lequel un utilisateur interagit avec l'articulateur numérique à l'aide de dispositifs d'entrée du dispositif informatique pour déplacer manuellement une mâchoire inférieure dans la simulation.

10. Système de traitement de données comprenant des moyens pour générer un plan de traitement dentaire comprenant :
un générateur de plan de traitement configuré pour déterminer un plan de traitement sur la base d'un profil de mâchoire du patient ;
le profil de mâchoire du patient comportant au moins une mesure de la taille de la mâchoire du patient ;
le générateur de plan de traitement comportant un modèle paramétrique de mouvement de mâchoire sur la base d'un ensemble de données de mouvement et de mesure de mâchoire et de denture humaines ;
le modèle paramétrique de mouvement de mâchoire étant divisé en une pluralité de segments représentatifs ; et
le générateur de plan de traitement étant configuré en outre pour comparer le profil de mâchoire du patient au modèle paramétrique de mouvement de mâchoire pour déterminer lequel des segments représentatifs est le plus similaire au profil de mâchoire du patient et pour générer le plan de traitement dentaire sur la base du segment représentatif identifié comme le plus similaire au profil de mâchoire du patient.

11. Système selon la revendication 10, dans lequel le générateur de plan de traitement est en outre configuré pour :
générer un modèle de restauration dentaire sur la base du segment représentatif qui est le plus similaire au profil de mâchoire du patient ;
identifier les interférences ; et
générer un plan de traitement dentaire modifié qui évite les interférences identifiées.

12. Système selon la revendication 10, dans lequel l'ensemble de données de mouvement et de mesure de mâchoire et de denture humaines inclut des modèles dentaires numériques tridimensionnels générés à partir de balayages et une limite d'interférence de prétraitement déterminée à partir de données de mouvement.

13. Système selon la revendication 10, comprenant en outre un générateur de limite d'interférence, dans lequel le générateur de limite d'interférence est configuré pour :
déterminer une limite d'interférence de pré-traitement sur la base du segment représentatif identifié comme le plus similaire au profil de mâchoire du patient ; et
fournir la limite d'interférence de pré-traitement au générateur de plan de traitement dentaire.

14. Système selon la revendication 13, dans lequel le générateur de plan de traitement est configuré pour déterminer en outre le plan de traitement sur la base de la limite d'interférence de prétraitement.
